Europäisches Patentamt

European Patent Office

Office européen des brevets

(11) Veröffentlichungsnummer: **0 337 289**
**A1**

# (12) EUROPÄISCHE PATENTANMELDUNG

(21) Anmeldenummer: 89106035.2

(22) Anmeldetag: 06.04.89

(51) Int. Cl.⁴: **A61K 31/12 , A61K 47/00**

(30) Priorität: **10.04.88 DE 3811936**

(43) Veröffentlichungstag der Anmeldung:
**18.10.89 Patentblatt 89/42**

(84) Benannte Vertragsstaaten:
**ES GR**

(71) Anmelder: **Minninger, Konrad**
**Hattinger Strasse 45**
**D-5830 Schwelm(DE)**

(72) Erfinder: **Minninger, Konrad**
**Hattinger Strasse 45**
**D-5830 Schwelm(DE)**
Erfinder: **Tang, David, Dr.**
**Hauptstrasse 304**
**D-4690 Herne 2(DE)**
Erfinder: **Oberhagemann, Rainer**
**Hauptstrasse 304**
**D-4690 Herne 2(DE)**

(74) Vertreter: **Patentanwaltsbüro Cohausz &**
**Florack**
**Postfach 14 01 20 Schumannstrasse 97**
**D-4000 Düsseldorf 1(DE)**

(54) Dithranol-haltiges pharmazeutisches Produkt gegen schuppende Hauterkrankungen.

(57) Das Dithranol-haltige pharmazeutische Produkt zur lokalen Therapie von schuppenden Hauterkrankungen enthält auf 100 g des Produktes 0,005 g bis 1 g Dithranol, gelöst in der Lösungsmittelkombination von 1 bis 5 g eines aliphatischen 1,2-Alkandiols mit 2 bis 4 Kohlenstoffatomen und 98,995 g bis 94 g eines Glycerintriesters und/oder 1,2-Propandioldiesters einer oder mehrerer Fettsäuren. Das Dithranol-haltige pharmazeutische Produkt enthält gegebenenfalls zusätzlich Salicylsäure, so daß das Produkt auf 100 g desselben 0,005 bis 1 g Dithranol und 0,1 bis 5 g Salicylsäure, gelöst in der Lösungsmittelkombination von 1 bis 5 g des aliphatischen 1,2-Alkandiols mit 2 bis 4 Kohlenstoffatomen und 98,895 bis 89 g des Glycerintriesters und/oder des 1,2-Propandioldiesters einer oder mehrerer Fettsäuren enthält. Das pharmazeutische Produkt enthält gegebenenfalls Steinkohleteer oder Steinkohleteerdestillat in pharmazeutisch reiner Form mit einem Gewichtsanteil von 1 bis 5 g pro 100 g des Produktes.

## Dithranol-haltiges pharmazeutisches Produkt gegen schuppende Hauterkrankungen

Die Erfindung betrifft ein Dithranol als Wirkstoff enthaltendes pharmazeutisches Produkt zur lokalen Therapie von schuppenden Hauterkrankungen.

Zu diesen Erkrankungen zählen beispielsweise Psoriasis vulgaris, Psoriasis capitis, das seborrhoeische Ekzem, Tinea amiantacea und Seborrhoea sicca sowie andere schuppende Haut- und Kopferkrankungen, wie z.B. Pityriasis capillitii simplex. Das Mittel ist auch zur Behandlung von Alopecia areata geeignet.

Es ist beispielsweise aus Ring, Fröhlich, Wirkstoffe in der dermatologischen Therapie, Springer Verlag (1985), Seite 85 bekannt, daß Dithranol zur Behandlung solcher schuppenden Hauterkrankungen verwendet wird. Dieser Wirkstoff wirkt zytostatisch und verlangsamt dadurch die Zellteilung. Bis jetzt wird er in Form von Salben, Cremes, Pasten oder Stiften angewandt.

Es hat sich herausgestellt, daß die vorgenannten Dithranol-haltigen Salben, Cremes oder Pasten in der Handhabung nicht immer günstig sind. Die auf dem Markt befindlichen Mittel enthalten bis zu 5% Dithranol in fester Form und haben den Nachteil, daß der Wirkstoff Dithranol nicht in einer Form vorliegt, der für die Anwendung optimal ist. Daher finden diese Produkte vor allem im Langzeit gebrauch, insbesondere bei der Behandlung der Kopfhaut bei den Patienten nur eingeschränkte Akzeptanz. Außerdem sind bei der Form, in der der Wirkstoff in diesen bekannten Produkten vorliegt, höhere Konzentrationen nötig, um einen Abheilungserfolg herbeizuführen.

Die Aufgabe der Erfindung besteht darin, ein Dithranol-haltiges pharmazeutisches Produkt der eingangs genannten Art zu schaffen, das bei guter Verträglichkeit über längere Zeiträume ohne nachteilige Wirkung für die Patienten ist, eine nicht stationäre Behandlung ermöglicht und eine optimale Patienten-Mitarbeit gewährleistet.

Erfindungsgemäß wird diese Aufgabe dadurch gelöst, daß eine besondere Kombination von organischen Lösungsmitteln in besonderem Mengenverhältnis zueinander und zum Wirkstoff für den Wirkstoff Dithranol verwendet wird, so daß der schwerlösliche Wirkstoff in dem erfindungsgemäßen Produkt nicht nur gelöst vorliegt, sondern die erfindungsgemäßen pharmazeutischen Produkte auch über lange Zeit haltbar sind und keine das pharmazeutische Produkt verfärbende Zersetzungsprodukte des Dithranols bilden. Das erfindungsgemäße pharmazeutische Produkt enthält auf 100 g des Produkts 0,005 bis 1 g Dithranol, 1 bis 5 g eines niederaliphatischen 1,2-Alkandiols mit 2 bis 4 Kohlenstoffatomen und 94 g bis 98,995 g eines Glycerintriesters und/oder eines 1,2-Propandioldiesters einer oder mehrerer Fettsäuren mit gesättigtem oder ungesättigtem Kohlenwasserstoffrest.

Überraschenderweise wurde gefunden, daß eine optimale Wirkung erreicht werden kann, wenn der Wirkstoff Dithranol in molekular gelöster Form im Präparat vorliegt. Dadurch wird möglich, mit wesentlich niedrigeren Konzentrationen einen Abheilungserfolg mit weniger Nebenwirkungen zu erzielen.

Die erfindungsgemäßen pharmazeutischen Produkte zeichnen sich durch bestmögliche Wirkstoffliberation bei optimaler biologischer Verfügbarkeit und maximaler Pharmakokinetik durch die Anwendung in molekular gelöster Form aus.

Die erfindungsgemäßen pharmazeutischen Produkte sind auch bei der Langzeitanwendung gut verträglich und haltbar. Bisher wurden keine allergischen Reaktionen beobachtet. Dabei wird eine hohe Wirksamkeit erreicht.

Unter den in der erfindungsgemäßen Kombination von organischen Lösungsmitteln verwendeten niederaliphatischen 1,2-Alkandiolen mit 2 bis 4 Kohlenstoffatomen haben sich das 1,2-Propandiol und die verschiedenen Butylenglykole mit benachbarten Hydroxylgruppen als besonders günstig erwiesen. Dabei ist ganz besonders bevorzugt 1,2-Propandiol.

Unter den in der erfindungsgemäßen Kombination von organischen Lösungsmitteln eingesetzten Glycerintriester und/oder 1,2-Propandioldiestern einer Fettsäure haben sich als besonders günstig erwiesen die Glycerintriester und 1,2-Propandioldiester mittelkettiger Fettsäuren wie Caprylsäure, Caprinsäure oder deren Mischungen. Gute Ergebnisse werden jedoch auch mit Estern von Fettsäuren pflanzlicher Öle erhalten. Auch sind brauchbar pflanzliche Öle selbst, die hauptsächlich Triglyceride gesättigter und ungesättigter Fettsäuren darstellen. Auch sind anstelle der Glycerintriester oder 1,2-Propandioldiester Mischungen dieser Ester mit anderen Estern von ein- oder zweiwertigen Alkoholen mit insbesondere mittelkettigen Fettsäuren einsetzbar.

Eine weitere Verbesserung der Wirkung der erfindungsgemäßen pharmazeutischen Produkte läßt sich dadurch erzielen, daß sie zusätzlich Salicylsäure enthalten. Zwar ist bekannt (E. Mutschler, Arzneimittelwirkungen, Wissenschaftliche Verlagsgesellschaft (1975) Seite 373), daß Salicylsäure die Ablösung von Schuppen und die Erweichung von Hornmaterial bewirkt, wobei die Salicylsäure ebenfalls in Form von Salben, Cremes oder Pasten eingesetzt werden kann. Überraschenderweise wird die Löslichkeit des Dithranols durch die Salicylsäure, selbst bei größeren Konzentrationen des einen oder anderen oder der Wirkstoffe in der erfindungsgemäßen Kombination organischer Lösungsmittel nicht beeinträchtigt. Die Haltbarkeit des Wirkstoffs Dithranol gegen Oxydation in dem erfindungsgemäßen Produkt wird durch den gewählten Träger in Form eines Öls und wird zusätzlich bei gleichzeitiger Anwesenheit von Salicylsäure sogar erhöht. Durch die Salicylsäure wird eine höhere Penetration durch Keratolyse und Abschuppung bewirkt.

Die sowohl Dithranol als auch Salicylsäure enthaltenden pharmazeutischen Produkte bestehen, bezogen auf 100 g des pharmazeutischen Produktes, aus 0,005 bis 1 g Dithranol, 0,1 bis 5 g Salicylsäure, 1 bis 5 g des niederaliphatischen 1,2-Alkandiols mit 2 bis 4 Kohlenstoffatomen und 89 bis 98,895 g des Glycerintriesters und/oder 1,2-Propandioldiesters von Fettsäuren, insbesondere der mittelkettigen Fettsäuren, ganz besonders von Caprylsäure, Caprinsäure oder Mischungen hiervon. Besonders gute Ergebnisse werden mit den 1,2-Propandioldiestern von Caprylsäure, Caprinsäure oder Mischungen hiervon erzielt. Prinzipiell werden dabei bei steigendem Gehalt der Produkte an Salicylsäure steigende Mengen an 1,2-Propandiol eingesetzt.

Eine wichtige Ursache für die hohe Wirksamkeit der erfindungsgemäßen pharmazeutischen Produkte liegt möglicherweise darin, daß die gewählte Kombination von organischen Lösungsmitteln im besonderen Mengenverhältnis zueinander und zu den Wirkstoffen in therapeutisch besonders günstiger Weise auf die erkrankte Haut einwirkt und dadurch einen sehr wirksamen Träger bildet, der das Dithranol und, falls ebenfalls vorhanden, auch die Salicylsäure in besonders optimaler Form an seinen Wirkungsort bringt. Weiterhin ist die Behandlung mit den erfindungsgemäßen pharmazeutischen Produkten nicht mit unerwünschten und unakzeptablen Belastungen für die Patienten verbunden, weil die erfindungsgemäßen pharmazeutischen Produkte rasch und vollständig in der Haut resorbiert werden. Es wurde eine sehr hohe Patientenakzeptanz und Compliance erreicht.

Bevorzugte Ausführungsbeispiele der Erfindung werden nachstehend im Zusammenhang mit ihrer Herstellungsvorschrift erläutert. Die dafür eingesetzten Stoffe genügen den Reinheitsvorschriften des D.A.B..

Gemäß einer solchen bevorzugten Ausführungsform enthält das erfindungsgemäße pharmazeutische Produkt, sei es enthaltend Dithranol als einzigen Wirkstoff oder enthaltend sowohl Dithranol als auch Salicylsäure, zusätzlich Steinkohleteer oder Steinkohleteerdestillat in pharmazeutisch zulässiger Form in einer Menge von 1 bis 5 g pro 100 g des Produktes, möglichst in standardisierter Form. Dieser Zusatz dient zur Linderung des bisweilen auftretenden Erythems und/oder Juckreizes. Auch für diese zusätzliche Komponente ist die gewählte Kombination organischer Lösungsmittel eine besonders günstige Anwendungsform.

Zur Herstellung der erfindungsgemäßen pharmazeutischen Produkte wird beispielsweise eine abgewogene Menge von 0,005 bis 1 g Dithranol mit einer Menge von 1 bis 5 g des aliphatischen 1,2-Alkandiols mit 2 bis 4 Kohlenstoffatomen verrieben und die erhaltene Verreibung portionsweise mit 98,995 bis 94 g des jeweils verwendeten Esters versetzt und weiter verrieben oder gerührt, bis eine klare ölige Lösung entsteht.

Bevorzugt werden dabei für die meisten Anwendungsfälle 0,03 bis 0,75 g Dithranol, 2 g 1,2-Propandiol und 97,97 bis 97,25 g des jeweils verwendeten Esters, insbesondere 0,5 g Dithranol, 2 g 1,2-Propandiol und 97,5 g des jeweils verwendeten Esters. Bevorzugt wird dabei eingesetzt als Ester der 1,2-Propandioldiester von Caprylsäure oder Caprinsäure oder eines Gemischs hiervon, insbesondere im zuletzt genannten ganz besonders bevorzugten Fall. Dadurch wird bei guter Verträglichkeit und hoher Wirksamkeit die Beibehaltung der Erscheinungsfreiheit über eine vergleichsweise lange Dauer aufrechterhalten, insbesondere nach vorhergehender Abschuppung.

Beispielsweise besteht das. erfindungsgemäße pharmazeutische Produkt aus 0,5 g Dithranol, 5 g Salicylsäure, 2,0 g 1,2-Propandiol und 92,5 g des 1,2-Propandiolesters von Caprylsäure, Caprinsäure oder Mischungen hiervon.

Ein anderes erfindungsgemäßes Produkt besteht aus 0,5 g Dithranol, 5,0 g Salicylsäure, 2,0 g 1,2-Propandiol, 3,0 g Steinkohleteerdestillat und 89,5 g Propandioldiester von Caprylsäure, Caprinsäure oder Mischungen hiervon.

Das erfindungsgemäße pharmazeutische Präparat wird je nach vorherigem Erscheinungsbild auf die betreffenden Hautpartien aufgetragen. Je nach Konzentration des oder der Wirkstoffe in dem erfindungsgemäßen pharmazeutischen Präparat wird dieses über den erforderlichen Behandlungszeitraum bei einer täglichen Anwendungszeit von ca. 10 bis 60 Minuten oder mehr auf die betroffenen Hautpartien aufgetragen und dann abgewaschen. Eine solche Minuten-Therapie bei täglicher ambulanter Behandlung erfolgt während einer Woche, wobei am Wochenende keine Anwendung erfolgen muß.

Je nach Befund und Stärke des Krankheitsbildes kann die Behandlungsdauer von täglich 10 bis 60 Minuten oder mehr bei unterschiedlichen Konzentrationen gewählt werden.

Bei Anwendung auf der Kopfhaut bzw. im Gesichtsbereich wird um Nebenwirkungen zu vermeiden eine Konzentration von 0,03 % bzw. 0,025 % Dithranol bevorzugt verwendet, wobei die Anwendungsdauer auf mehrere Stunden bzw. über Nacht ausgedehnt wird.

Nach einer so durchgeführten Therapie, bei welcher eine steigende oder geringer werdende Konzentration angewandt wird, kann dann eine genaue Therapie für den jeweiligen Patienten zur optimalen Behandlung auch unter dem Aspekt der geringst möglichen Belastung bei keinen oder nur geringen Nebenwirkungen ermittelt werden.

Die therapeutische Anwendung von zwei Ausführungsbeispielen, die

zu a) 0,03 g Dithranol und

zu b) 0,5 g Dithranol

5 g Salicylsäure, 2,0 g 1,2-Propandiol und zu a) 92,7 g, zu b) 92,5 g des 1,2-Propandioldiesters von Caprylsäure, Caprinsäure oder Mischungen hiervon enthält, wird aufgrund einer Patientengruppe von ca. 150 Patienten, die an Psoriasis litten, erläutert.

Die Behandlung bestand darin, daß

a) das Produkt bei Erkrankungen der Kopfhaut ein- bis dreimal wöchentlich auf die vorher befallenen Stellen aufgetragen, und nach einer Einwirkungszeit von mind. 8 Stunden mit einem handelsüblichen medizinischen Haarwaschmittel ausgewaschen wird, und

b) bei Erkrankungen am Körper einmal täglich auf die vorher befallenen Stellen aufgetragen wird; nach ca. 10 bis 60 Minuten oder mehr wird das Öl mit einer rückfettenden Seife abgewaschen und danach die befallenen Stellen mit einer Vitamin A-Säure-Harnstoffsalbe nachbehandelt werden.

Zur Herstellung eines Kombinationsproduktes, bei dem die zytostatische Wirkung des Dithranols mit der schuppenlösenden Wirkung der Salicylsäure kombiniert wird, wird beispielsweise eine abgewogene Menge von 5 g Salicylsäure mit 2 g 1,2-Propandiol verrieben und die Verreibung wird unter Zugabe von 0,5 g Dithranol fortgesetzt. Anschließend werden unter fortdauerndem Verreiben und/oder Verrühren portionsweise 92,5 g eines Gemisches der 1,2-Propanglykoldiester von Caprylsäure, Caprinsäure oder Gemischen hiervon zugegeben bis eine klare ölige Lösung erreicht wird.

Das erfindungsgemäße pharmazeutische Produkt wurde von ca. 150 Patienten hervorragend akzeptiert. Dazu trägt sicher nicht zuletzt bei, daß das Produkt sehr rasch in die Haut einzieht, alle anderen Wirkstoffträger sind in der Handhabung - Auftragen, Wirkzeitraum, Abwaschen - wenig praktikabel.

Der wohl größte Vorteil des erfindungsgemäßen pharmazeutischen Produktes liegt sicher mit in der Tatsache begründet, daß eine problemlose Anwendung bei Kurzzeitwirkung, je nach Krankheitsbild in Minuten oder Stundentherapie bei optimaler Patientenakzeptanz und Compliance erfolgt.

Die vorstehend beschriebenen pharmazeutischen Produkte können außer zur unmittelbaren Behandlung der eingangs genannten schuppenden Hauterkrankungen mit sehr gutem Erfolg auch zur Nachbehandlung nach vorhergehender Abschuppung eingesetzt werden. Dadurch wird es möglich, die für die Patienten auch aus psychologischen Gründen so besonders wichtige Erscheinungsfreiheit über besonders lange Zeiten aufrechterhalten.

## Ansprüche

1. Dithranol als Wirkstoff enthaltendes pharmazeutisches Produkt zur lokalen Therapie von schuppenden Hauterkrankungen dadurch gekennzeichnet daß das pharmazeutische Produkt auf 100 g des Produktes 0,005 bis 1 g Dithranol, 1 bis 5 g eines aliphatischen 1,2-Alkandiols mit 2 bis 4 Kohlenstoffatomen und 98,995 bis 94 g eines Glycerintriesters und/oder 1,2-Propandioldiesters einer oder mehrerer Fettsäuren enthält.

2. Pharmazeutisches Produkt gemäß Anspruch 1, dadurch gekennzeichnet daß das aliphatische 1,2-Alkandiol 1,2-Propandiol oder ein Butylenglykol mit benachbarten Hydroxylgruppen ist.

3. Pharmazeutisches Produkt gemäß Anspruch 1 oder 2, dadurch gekennzeichnet daß der enthaltene Ester der einer oder mehrerer mittelkettiger Fettsäuren ist.

4. Pharmazeutisches Produkt gemäß Anspruch 3, dadurch gekennzeichnet daß die mittelkettigen Fettsäuren Caprylsäure, Caprinsäure oder ein Gemisch aus beiden Säuren ist.

5. Pharmazeutisches Produkt nach Anspruch 1 oder 2, dadurch gekennzeichnet daß der Ester ein pflanzliches fettes Öl ist.

6. Pharmazeutisches Produkt gemäß Anspruch 5, dadurch gekennzeichnet daß der Ester ein Glycerintriester einer langkettigen gesättigten oder ungesättigten Fettsäure ist.

7. Pharmazeutisches Produkt gemäß einem der Ansprüche 1 bis 6, dadurch gekennzeichnet daß es auf 100 g des Produktes 0,03 bis 0,75 g Dithranol, 2 g 1,2-Propandiol und 97,97 bis 97,25 g des Glycerintriesters oder 1,2-Propandioldiesters von Caprylsäure, Caprinsäure oder deren Mischungen enthält.

8. Pharmazeutisches Produkt gemäß Anspruch 7, dadurch gekennzeichnet daß es auf 100 g des Produktes 0,5 g Dithranol, 2 g 1,2-Propandiol und 97,5 g 1,2-Propandioldiesters von Caprylsäure, Caprinsäure oder deren Mischungen enthält.

9. Pharmazeutisches Produkt nach einem der Ansprüche 1 bis 6,
**dadurch gekennzeichnet** daß es auf 100 g des Produktes 0,005 bis 1 g Dithranol, 1 bis 5 g des aliphatischen 1,2-Alkandiols mit 2 bis 4 Kohlenstoffatomen, 0,1 bis 5 g Salicylsäure und 98,895 bis 89 g des Glycerintriesters oder des 1,2-Propandioldiesters einer oder mehrerer Fettsäuren enthält.

10. Pharmazeutisches Produkt gemäß Anspruch 7,
**dadurch gekennzeichnet** daß es auf 100 g des Produktes 0,03 bis 0,75 g Dithranol, 2 g 1,2-Propandiol, 0,1 bis 5 g Salicylsäure und 97,87 bis 92,25 g des Glycerintriesters oder 1,2-Propandioldiesters von Caprylsäure, Caprinsäure oder deren Mischungen enthält.

11. Pharmazeutisches Produkt gemäß Anspruch 8,
**dadurch gekennzeichnet** daß es auf 100 g des Produktes 0,5 g Dithranol, 2 g 1,2-Propandiol, 5 g Salicylsäure und 92,5 g des 1,2-Propandioldiesters von Caprylsäure, Caprinsäure oder deren Mischungen enthält.

12. Pharmazeutisches Produkt gemäß einem der Ansprüche 1 bis 11,
**dadurch gekennzeichnet** daß es auf 100 g des Produktes 1 bis 5 g Steinkohleteer oder eines Steinkohleteerdestillats in pharmazeutisch reiner Form enthält.

EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | GB-A-2 164 254 (L'OREAL) * Ansprüche 1,2,4,9; Seite 1, Zeilen 37-38,46-51; Seite 2, Zeilen 31-32,34-35 * --- | 1-5,7,9 ,10,12 | A 61 K 31/12 A 61 K 47/00 |
| Y | CHEMICAL ABSTRATCS, Band 97, Nr. 26, 27. Dezember 1982, Seite 438, Nr. 222884h, Columbus, Ohio, US; R.H. BRIEDE et al.: "Water-washable dithranol preparations", & PHARM. WEEKBL. 1982, 117(40), 937-8 * Zusammenfassung * ----- | 1-5,7,9 ,10,12 | |

RECHERCHIERTE SACHGEBIETE (Int. Cl.4)

A 61 K

Der vorliegende Recherchenbericht wurde für alle Patentansprüche erstellt

| Recherchenort | Abschlußdatum der Recherche | Prüfer |
|---|---|---|
| DEN HAAG | 27-06-1989 | SCARPONI U. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
............................................................
& : Mitglied der gleichen Patentfamilie, übereinstimmendes Dokument